# EUROPEAN PATENT SPECIFICATION

(11) **EP 3 994 242 B1**
(45) Date of publication and mention of the grant of the patent: **05.08.2026**
(21) Application number: 20756993.0
(22) Date of filing: 29.07.2020
(51) Int. Cl.: C12M 1/00, C12M 3/00, C12M 1/34

(54) **BIOLOGICAL COMPONENT CASSETTE, BIOLOGICAL COMPONENT KIT, AND BIOLOGICAL COMPONENT TREATMENT SYSTEM**
KASSETTE FÜR BIOLOGISCHE KOMPONENTEN, KIT FÜR BIOLOGISCHE KOMPONENTEN UND BEHANDLUNGSSYSTEM FÜR BIOLOGISCHE KOMPONENTEN
CASSETTE DE COMPOSANT BIOLOGIQUE, KIT DE COMPOSANT BIOLOGIQUE ET SYSTEME DE TRAITEMENT DE COMPOSANT BIOLOGIQUE

(30) Priority: 02.08.2019 JP 2019143075
(43) Date of publication of application: 11.05.2022
(73) Proprietor: TERUMO Kabushiki Kaisha, Tokyo 151-0072 (JP)
(72) Inventor: IGARASHI, Masatsugu, Fujinomiya-shi, Shizuoka 418-0004 (JP)
(74) Representative: TBK
(86) International application number: PCT/JP2020/029079
(87) International publication number: WO 2021/024880

(56) References cited:
- WO-A1-2018/051982
- DE-A1- 102017 106 402
- US-A1- 2011 187 388
- US-A1- 2016 186 123

## Description

### [Technical Field]

The present invention relates to a biological component cassette according to the preamble of claim 1, the features of which are known from document DE 10 2017 106 402 A1. Such a biological component cassette has flow paths through which a biological component flows. Moreover, the invention pertains to a biological component kit including the biological component cassette, and a biological component treatment system including the biological component kit and the biological component cassette.

### [Background Art]

In the practice of regenerative medicine, biological cells (biological components) are collected and cultured, and the cultured cells are administered to a patient. In a process of culturing cells, for example, as disclosed in Document JP 2017 - 143 775 A, a cell culture device (biological component treatment device), which is equipped with a cell culture container (bioreactor) having hollow fibers inside a case, is used. In such a biological component treatment device, a biological component kit having a plurality of medical bags and the bioreactor is set, and after a liquid containing cells is supplied into the hollow fibers and the cells are made to adhere to the interior of the hollow fibers, the cells are made to undergo proliferation by further delivering a culture medium into the cell culture container.

Document US 2011 / 0 187 388 A1 discloses a single-use biomass sensing device having a chip configured to change its color upon contacting a biological component in a liquid. The chip is immersed into the liquid for detection of the biological component.

Document WO 2018 / 051 982 A1 discloses a blood component sampling cassette made of a flexible material.

### [Summary of Invention]

propriately controlling the growth condition of the cells (the number of cells, etc.).

Therefore, it is desirable to detect the parameters of the culture medium supplied to the bioreactor in real time, and to appropriately adjust the supply amount and the rate at which the culture medium is supplied.

The present invention has been devised in relation to the aforementioned technique, and has the object of providing a biological component cassette, a biological component kit, and a biological component treatment system, which enable setting to be carried out efficiently, and which are capable of stabilizing product quality by enabling easy detection of parameters related to culturing of cells.

In order to achieve the aforementioned object, a first aspect of the present invention is a biological component cassette having therein a flow path through which a liquid for culturing cells is allowed to flow, and including a cassette main body formed in a sheet shape that possesses flexibility, wherein in the flow path, there is provided a target parameter detection part through which a parameter related to culturing of cells is detectable, and the target parameter detection part includes a chip configured to undergo coloring in response to a predetermined substance contained in the liquid, wherein the target parameter detection part includes a bulging portion that protrudes in a direction perpendicular to a planar direction of the cassette main body, the bulging portion being configured to accommodate the chip therein.

Further, in order to achieve the aforementioned object, a second aspect of the present invention is a biological component kit including a tube through which a liquid for culturing cells is allowed to flow, and a biological component cassette to which the tube is connected, wherein the biological component cassette includes therein a flow path through which the liquid is allowed to flow, and includes a cassette main body formed in a sheet shape that possesses flexibility, in the flow path, there is provided a target parameter detection part through which a parameter related to culturing of cells is detectable, and the target parameter detection part includes a chip configured to undergo coloring in response to a predetermined substance contained in the liquid.

Further, in order to achieve the aforementioned object, a third aspect of the present invention is a biological component treatment system, including a biological component kit including a tube through which a liquid for culturing cells is allowed to flow, and a biological component cassette to which the tube is connected, and a biological component treatment device in which the biological component kit is set, wherein the biological component cassette includes therein a flow path through which the liquid is allowed to flow, and includes a cassette main body formed in a sheet shape that possesses flexibility, in the flow path, there is provided a target parameter detection part through which a parameter related to culturing of cells is detectable, the target parameter detection part includes a chip configured to undergo coloring in response to a predetermined substance contained in the liquid, and the biological component treatment device includes an optical sensor configured to detect the parameter related to culturing of cells, by emitting measurement light toward the fluorescent chip and receiving excitation light from the chip.

In the above-described biological component cassette, the biological component kit, and the biological component treatment system, setting can be carried out efficiently, and it is possible to stabilize product quality by enabling easy detection of parameters related to culturing of cells.

### Brief Description of Drawings

[fig.1]FIG. 1 is a perspective view showing a biological component treatment system to which a biological component cassette and a biological component kit according to an embodiment of the present invention are applied;
[fig.2]FIG. 2 is an exploded perspective view of the biological component cassette;
[fig.3]FIG. 3 is a plan view showing a cassette main body and a peripheral portion thereof;
[fig.4]FIG. 4 is an explanatory diagram schematically showing liquid paths of the biological component kit at a time of cell culturing;
[fig.5]FIG. 5A is a block diagram showing an outline of a culture parameter detection unit, and FIG. 5B is a side cross-sectional view showing the culture parameter detection unit in an enlarged manner;
[fig.6]FIG. 6A is an exploded perspective view showing a laminated structure of a first exemplary configuration of a fluorescent chip, and FIG. 6B is an exploded perspective view showing a laminated structure of a second exemplary configuration of a fluorescent chip; and
[fig.7]FIG. 7 is a flowchart showing a process of manufacturing the cassette main body including the fluorescent chip.

### Description of Embodiments

Preferred embodiments of the present invention will be presented and described in detail below with reference to the accompanying drawings.

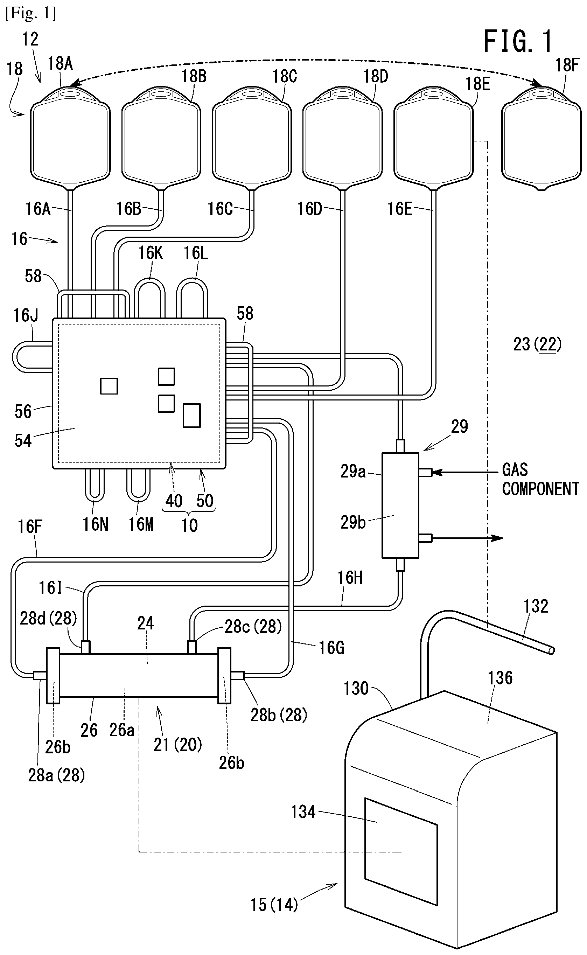

A biological component cassette 10 (hereinafter, simply referred to as a cassette 10) according to an embodiment of the present invention, as shown in FIG. 1, constitutes one part of a biological component kit 12 (hereinafter, simply referred to as a kit 12), and is set in a biological component treatment device 14. The cassette 10 collects together a plurality of paths of the kit 12, and is used as a structural body through which a liquid containing a biological component and a liquid for processing of the biological component are capable of flowing (being circulated).

The kit 12 includes, as members that constitute the plurality of paths, and in addition to the cassette 10, a plurality of tubes 16, a plurality of medical bags 18, and a treatment unit 20 in which processing is performed in the biological component treatment device 14. The kit 12 allows a plurality of types of liquids contained in each of the medical bags 18 to flow through the cassette 10 and through each of the tubes 16 under the operation of the biological component treatment device 14, and is constituted so as to obtain a target product by processing the liquids in the treatment unit 20.

The kit 12 according to the present embodiment is a cell proliferation kit which is used in a cell expansion or proliferation process for expanding biological cells (biological components) in regenerative medicine, and a bioreactor 21 in which the cells are seeded and expanded is applied to the treatment unit 20. Further, as the liquids that flow inside the kit 12, there may be cited a solution containing cells (hereinafter referred to as a cell solution), a culture medium (culture solution) which is supplied in order to expand or grow the cells, a cleaning solution for cleaning the interior of the kit 12, and a releasing solution for releasing the cells. More specifically, the kit 12 and the biological component treatment device 14 constitute a biological component treatment system 22 that seeds the bioreactor 21 with the cell solution, and that supplies the culture medium to thereby culture the cells, and thereafter, releases and collects the expanded cells from the bioreactor 21. Hereinafter, the biological component treatment device 14 may also be referred to as a cell expansion device 15, and the biological component treatment system 22 may also be referred to as a cell expansion system 23.

The biological cells are not particularly limited, and may include, for example, cells (T cells and the like) contained in blood, and stem cells (ES cells, iPS cells, mes-enchymal stem cells, and the like). An appropriate culture medium may be selected according to the biological cells, and for example, as such a culture medium, there may be cited a balanced salt solution (BSS) as a basic solution, and various amino acids, vitamins, serum and the like may be added thereto in order to prepare the culture medium. Further, the cleaning solution is not particularly limited, and as examples thereof, there may be cited buffering solutions such as PBS (Phosphate Buffered Salts), TBS (Tris-Buffered Saline) and the like, or physiological saline. Further, as the releasing solution, for example, trypsin or an EDTA solution can be applied.

Among the plurality of medical bags 18 of the kit 12, there are included a cell solution bag 18A in which the cell solution is accommodated, a cleaning solution bag 18B in which the cleaning solution is accommodated, and a culture medium bag 18C in which the culture medium is accommodated. Furthermore, as the plurality of medical bags 18, the kit 12 includes empty bags, and such empty bags include a waste liquid bag 18D into which a liquid that is discarded in the cell expansion process flows, and a collection bag 18E in which cells (and other liquids) obtained in the expansion process are collected. Further, among the medical bags 18, a releasing solution bag 18F in which the releasing solution is accommodated is separately prepared. During the course of the expansion process, the releasing solution bag 18F is exchanged by the operator with one of the medical bags 18 (for example, the cell solution bag 18A) that has been connected beforehand.

The cell solution bag 18A, the cleaning solution bag 18B, and the culture medium bag 18C, etc., are aseptically joined to ends of the respective tubes 16 using a non-illustrated aseptic joining device (sterile tubing welder). Alternatively, each of the medical bags 18 may be fixed to ends of the respective tubes 16 in a non-separable manner, and may have a structure for ensuring sterility inside the kit 12. Further, alternatively, the kit 12 may apply a connection structure (not shown) that enables a detachable connection between the tubes 16 and each of the medical bags 18.

Although not particularly limited, for the bioreactor 21 of the kit 12, it is preferable to use a culture medium substrate having a large surface area, and for example, a structure having hollow fibers 24 may be applied thereto. More specifically, the bioreactor 21 includes a plurality of the hollow fibers 24 (for example, ten thousand or greater), and a cylindrical container 26 having a main space 26a therein in which the plurality of hollow fibers 24 are accommodated.

The plurality of hollow fibers 24 include internal cavities (not shown) that penetrate along the direction of extension thereof, and the cells are cultured by becoming adhered on inner peripheral surfaces of the hollow fibers 24 that constitute the internal cavities. The hollow fibers 24 are accommodated along an axial direction of the container 26, and both ends thereof are retained by non-illustrated retaining walls. The diameters of the hollow cavities, for example, are formed on the order of approximately 200 micrometers, and communicate with end spaces 26b on both axial sides of the retaining walls.

Further, the hollow fibers 24 include a plurality of non-illustrated pores therein that enable communication between the outer side (the main space 26a) and the inner cavities of the hollow fibers 24. The pores are formed with sizes that do not allow cells and proteins to pass therethrough, but on the other hand enable solutions and substances of low molecular weight to pass therethrough. The diameter of the pores is set, for example, on the order of 0.005 to 10 micrometers. Consequently, the culture medium, a predetermined gas component, and the like are supplied via the pores to the cells that are adhered to the inner peripheral surfaces of the hollow fibers 24. Hereinafter, a configuration in which liquid is primarily circulated in the inner cavities of the hollow fibers 24 may also be referred to as an IC (intra capillary) configuration, and a configuration in which liquid is primarily circulated on outer sides of the hollow fibers 24 may also be referred to as an EC (extra capillary) configuration.

The material constituting the hollow fibers 24 is not particularly limited, and as examples thereof, there may be cited polyolefin resins such as polypropylene, polyethylene and the like, and polymer materials such as polysulfone, polyether sulfone, polyacrylonitrile, polytelorafluoroethylene, polystyrene, polymethylmethacrylate, cellulose acetate, cellulose triacetate, regenerated cellulose, and the like.

The container 26 has an axial length which is capable of accommodating the hollow fibers 24 in a state that the hollow fibers are extended in a substantially linear shape. The container 26 is equipped with four terminals 28 (a first IC terminal 28a, a second IC terminal 28b, a first EC terminal 28c, and a second EC terminal 28d) that are connected respectively to the tubes 16. The first IC terminal 28a is provided at one end of the container 26 and communicates with the end space 26b on one end side. The second IC terminal 28b is provided at another end of the container 26 and communicates with the end space 26b on another end side. The first EC terminal 28c is provided on an outer peripheral surface of the container 26 in the vicinity of the other end side, and communicates with the main space 26a at a location in proximity to the other end. The second EC terminal 28d is provided on an outer peripheral surface of the container 26 in the vicinity of the one end side, and communicates with the main space 26a at a location in proximity to the one end.

The plurality of tubes 16 of the kit 12 include a cell solution tube 16A connected between the cell solution bag 18A and the cassette 10, a cleaning solution tube 16B connected between the cleaning solution bag 18B and the cassette 10, a culture medium tube 16C connected between the culture medium bag 18C and the cassette 10, a waste liquid tube 16D connected between the waste liquid bag 18D and the cassette 10, a collection tube 16E connected between the collection bag 18E and the cassette 10, a first IC tube 16F connected between the first IC terminal 28a of the bioreactor 21 and the cassette 10, a second IC tube 16G connected between the second IC terminal 28b of the bioreactor 21 and the cassette 10, a first EC tube 16H connected between the first EC terminal 28c of the bioreactor 21 and the cassette 10, and a second EC tube 16I connected between the second EC terminal 28d of the bioreactor 21 and the cassette 10.

A gas exchanger 29 that mixes a predetermined gas component with a liquid (the culture medium) is provided at an intermediate position of the first EC tube 16H. As an example of the gas component to be mixed, there may be cited a gas component that approximates the mixing ratio of natural air (nitrogen N₂: 75%, oxygen O₂: 20%, and carbon dioxide CO₂: 5%).

The structure of the gas exchanger 29 is not particularly limited, and in the same manner as the bioreactor 21, a structure can be applied in which a plurality of hollow fibers 29b are provided inside a container 29a. More specifically, the gas exchanger 29 guides the liquid flowing through the first EC tube 16H, into the inner cavities of the hollow fibers 29b, and during movement thereof inside the hollow fibers 29b, the gas component that is supplied to the interior of the container 29a (the space on the outer side of the hollow fibers 29b) is mixed with the liquid through the pores of the hollow fibers 29b.

In addition, by joining the aforementioned tubes 16 in advance, the cassette 10, which is one component of the kit 12, functions as a relay unit through which the cell solution, the cleaning solution, the culture solution, and the releasing solution of the respective medical bags 18 are allowed to flow to a different medical bag 18 or to the bioreactor 21. When the kit 12 is set in the cell expansion device 15, the cassette 10 is mounted in a cassette setting location inside the cell expansion device 15, which simplifies the wiring operation of the tubes 16 in the cell expansion process.

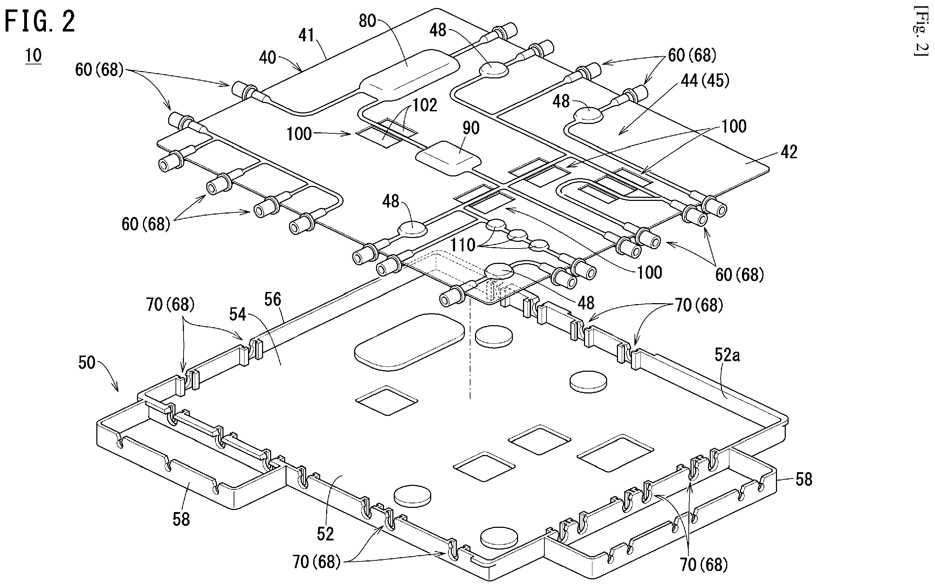

As shown in FIG. 2, the cassette 10 according to the present embodiment includes a soft cassette main body 40 to which the plurality of tubes 16 are directly connected, and a rigid frame 50 that retains the cassette main body 40 and is fixed to the cell expansion device 15.

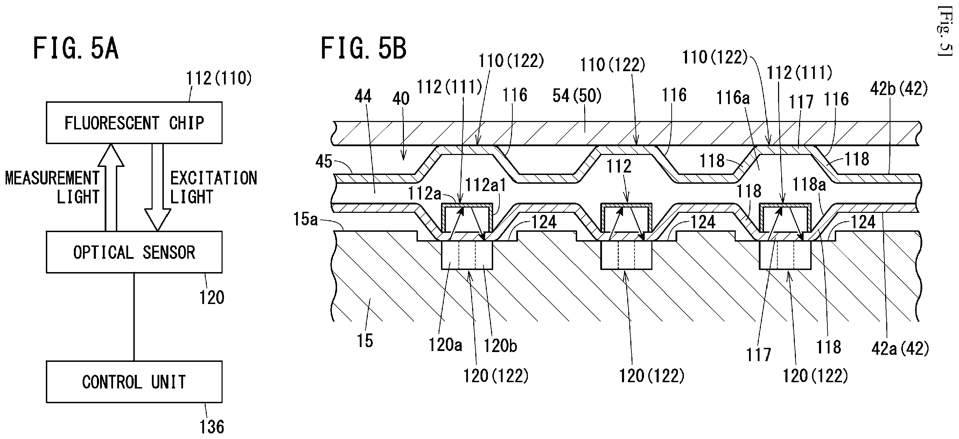

The cassette main body 40 exhibits a substantially rectangular shape, and is formed in a thin sheet shape which possesses flexibility. The cassette main body 40 is formed by stacking and joining (fusion bonding) together two resin sheets 42 made of a resin material in a thickness direction. In the fusion bonding of the pair of resin sheets 42, gas is supplied to and discharged between the pair of resin sheets 42 along grooves that are formed in a fusion bonding mold, whereby flow path walls 45, in which the resin sheets 42 are raised and protrude with semicircular shapes in cross-section, and flow paths 44 are formed on the inner sides thereof (refer also to FIG. 5B). The material constituting the resin sheets 42 is not particularly limited, insofar as it possesses flexibility that is capable of being deformed by the pressure of the liquids, and for example, a vinyl chloride resin, a polyolefin resin, a polyurethane resin, or the like may be applied thereto. An embossing process may be implemented on the surface of the cassette main body 40, and fine convex/concave irregularities may be formed therein. A plurality of connectors 60 for connection between the plurality of tubes 16 and the flow paths 44 are provided on outer edges 41 of the cassette main body 40.

On the other hand, the frame 50 is constituted by a resin material that is harder (having a greater modulus of elasticity) than the cassette main body 40, and is formed in a thin recessed shape having an accommodation space 52 therein in which the cassette main body 40 is accommodated. The constituent material of the frame 50 is not limited to any particular material, however, there may preferably be used a thermoplastic resin material, for example, polypropylene, polycarbonate, polyamide, polysulfone, polyarylate, methacrylate-butylene-styrene copolymer, or the like.

The frame 50 includes a substantially rectangular shaped cover portion 54 which is slightly larger than the cassette main body 40, and side portions 56 that protrude a short distance from the outer periphery of the cover portion 54 in a direction perpendicular to the cover portion 54. The side portions 56 extend around the entire outer periphery of the cover portion 54. In the frame 50, the accommodation space 52 is opened through an opening 52a surrounded by the side portions 56 on an opposite side from the cover portion 54, thereby causing one surface of the cassette main body 40 to be exposed. Further, the frame 50 includes a retaining frame 58 that extends outward from each of the upper side and the right side of the side portions 56 and that retains the tubes 16 which are separated a predetermined distance from the side portions 56. Engaging portions 70 in which the respective connectors 60 are arranged and retained therein are provided in the side portions 56 at positions corresponding to the respective connectors 60 of the cassette main body 40. The connectors 60 and the engaging portions 70 constitute engagement mechanisms 68 for engagement with the cassette main body 40.

The aforementioned engagement mechanisms 68 are disposed respectively on four sides of the substantially rectangular shaped cassette 10. Consequently, the frame 50 retains the sheet-shaped cassette main body 40 in a stretched state, and suitably causes the flow paths 44 to be extended along a planar direction.

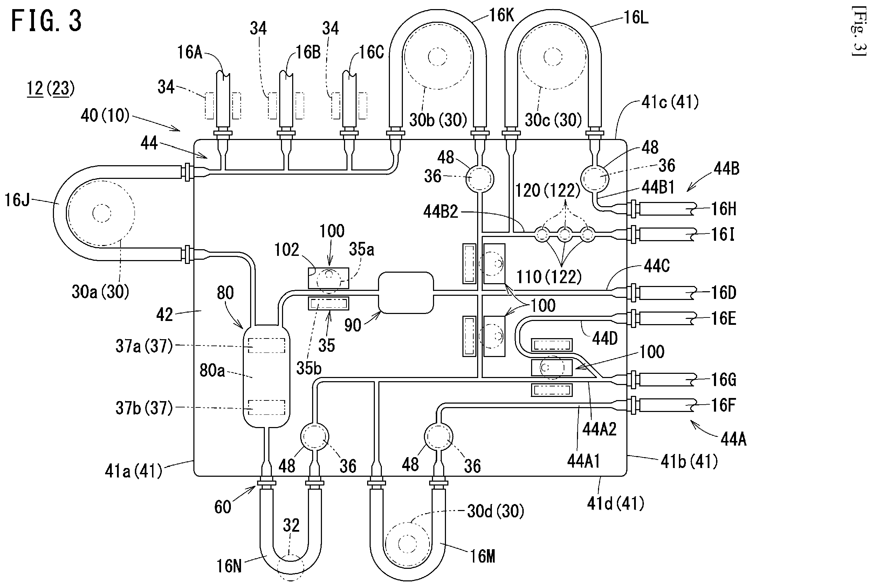

In addition, the cassette 10 is set inside the cell expansion device 15 in a state where the cassette main body 40 and the frame 50 are integrated, and the planar direction of the cassette main body 40 is set in an upright posture along the direction of gravity (in the vertical direction). More specifically, in the interior of the cell expansion device 15, the cassette 10 is fixed to the cassette setting location of the cell expansion device 15 while being oriented in the vertical direction shown in FIG. 3. Moreover, the cassette 10 in FIG. 3 is shown in a posture as viewed from the side of the cover portion 54 (the side of a touch panel 134 of the cell expansion device 15) in a state of being mounted in the cell expansion device 15, and in order to simplify description, the frame 50 is omitted and only the cassette main body 40 is shown.

More specifically, the outer edges 41 of the cassette main body 40 are constituted by a first short side 41a (left side in the figure), a second short side 41b (right side in the figure), a first long side 41c (upper side in the figure), and a second long side 41d (lower side in the figure). The cell solution tube 16A, the cleaning solution tube 16B, and the culture medium tube 16C are connected to the first long side 41c. The waste liquid tube 16D, the collection tube 16E, the first IC tube 16F, the second IC tube 16G, the first EC tube 16H, and the second EC tube 16I are connected to the second short side 41b.

Further, in the cell expansion system 23, in the set state, four pumps 30 are arranged at positions in proximity to the sides of the cassette 10. More specifically, in the set state, the cell expansion device 15 includes a first pump 30a disposed in proximity to the first short side 41a, a second pump 30b and a third pump 30c disposed in proximity to the first long side 41c, and a fourth pump 30d disposed in proximity to the second long side 41d.

In the kit 12 (cassette 10), as closed tubes 16 corresponding to the first to fourth pumps 30a to 30d, the first pump tube 16J is connected to the first short side 41a, the second pump tube 16K and the third pump tube 16L are connected to the first long side 41c, and the fourth pump tube 16M is connected to the second long side 41d. The first to fourth pump tubes 16J to 16M are arranged in a manner so that the portions thereof that are folded back in an arcuate shape are wrapped around circular shaped wound portions of the first to fourth pumps 30a to 30d.

For example, by being rotated so as to squeeze the respective pump tubes 16J to 16M wrapped therearound, the first to fourth pumps 30a to 30d apply a fluid force to the liquids inside the pump tubes 16J to 16M. The first pump 30a causes the liquid to flow in an IC route 44A to be described later, and the second pump 30b causes the liquid to flow in an EC route 44B to be described later. Further, the third pump 30c circulates the liquid of the EC route 44B, and the fourth pump 30d circulates the liquid of the IC route 44A.

In the cell expansion system 23, in the set state, an air bubble sensor 32 is arranged at a position in the vicinity of the second long side 41d of the cassette main body 40. Therefore, in the kit 12, a sensor tube 16N in the form of a closed tube 16 is connected to the second long side 41d, and in the set state, the sensor tube 16N is arranged so as to face the air bubble sensor 32.

Furthermore, in the cell expansion system 23, in the set state, outer side clamps 34 are arranged respectively on the cell solution tube 16A, the cleaning solution tube 16B, and the culture medium tube 16C. The outer side clamps 34 open and close the respective flow paths of the tubes 16 under the control of the cell expansion device 15. Further still, in the set state, the cell expansion system 23 includes a plurality of inner side clamps 35 that open and close predetermined flow paths 44 provided in the cassette main body 40.

As noted previously, the flow paths 44 having predetermined shapes are formed inside the outer edges 41 of the cassette main body 40, and extend along the planar direction. Further, the cassette main body 40 has, provided on the sheets, a plurality of pressure detection parts (detected parts for pressure detection) 48 in communication with the flow paths 44, a liquid level detection part (a detected part for liquid level detection) 80, a check valve unit 90, and a plurality of flow path opening/closing units 100 and a plurality of target parameter detection parts (detected parts for target parameter detection) 110 configured together with the flow paths 44.

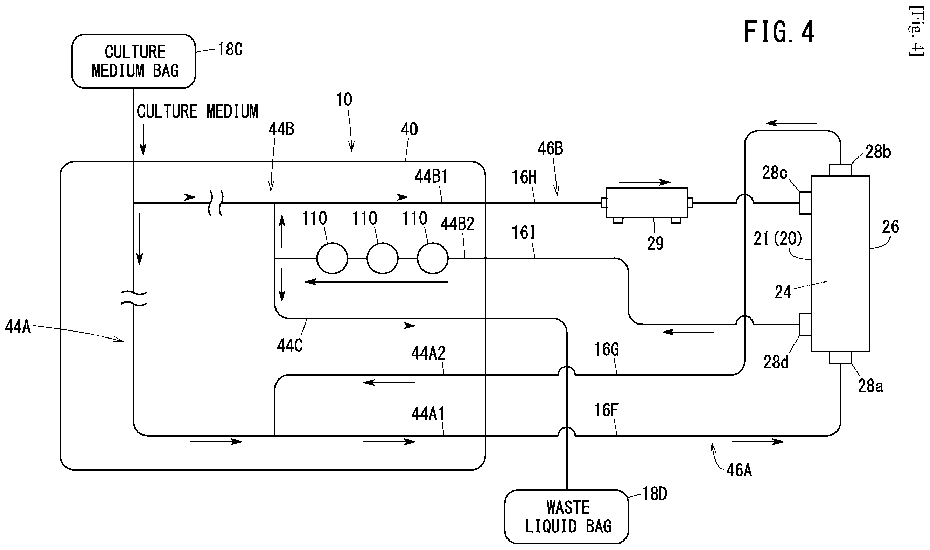

Although detailed description thereof is omitted, the flow paths 44 are constituted by an IC route 44A for supplying liquid to the inner cavities of the hollow fibers 24 together with the first and second IC tubes 16F and 16G, and an EC route 44B for supplying liquid to the outer side (the main space 26a) of the hollow fibers 24 together with the first and second EC tubes 16H and 16I. In particular, when the culture medium is supplied to the bioreactor 21, each of the plurality of outer side clamps 34 and the plurality of inner side clamps 35 is appropriately opened or closed, whereby the IC route 44A and the EC route 44B are placed in the state that is shown schematically in FIG. 4.

The IC route 44A includes inside the cassette main body 40 a first IC port path 44A1 in communication with the first IC tube 16F, and a second IC port path 44A2 in communication with the second IC tube 16G. In addition, in the IC route 44A, a circulation circuit 46A is formed that circulates liquid between the bioreactor 21 (inside the hollow fibers 24), the first and second IC port paths 44A1 and 44A2, and the first and second IC tubes 16F and 16G.

The EC route 44B includes inside the cassette main body 40 a first EC port path 44B1 in communication with the first EC tube 16H, and a second EC port path 44B2 in communication with the second EC tube 16I. In addition, in the EC route 44B, a circulation circuit 46B is formed that circulates liquid between the bioreactor 21 (outside the hollow fibers 24), the first and second EC port paths 44B1 and 44B2, and the first and second EC tubes 16H and 16I.

Returning to FIG. 3, the pressure detection parts 48 are provided respectively on downstream sides of the pumps 30 in the IC route 44A and the EC route 44B. The pressure detection parts 48, by being arranged so as to face toward pressure sensors 36 provided in the cell expansion device 15, make it possible to detect the pressures in the flow paths 44. Further, the liquid level detection part 80 is provided in the IC route 44A, temporarily stores the flowing liquid in a storage space 80a, and causes the liquid to flow out to the IC route 44A, together with causing gas to flow out to a route that differs from the route for the liquid. The liquid level detection part 80 is arranged so as to face toward liquid level sensors 37 (an upper sensor 37a and a lower sensor 37b) provided in the cell expansion device 15, and makes it possible to detect a liquid level of the liquid that is stored in the interior thereof. The check valve unit 90 is provided in the route from which the gas is allowed to flow out, and restricts inflowing of gas or liquid into the liquid level detection part 80 from the route.

The flow path opening/closing units 100 are constituted from a plurality of notches 102, and are disposed respectively in the plurality of inner side clamps 35 of the cell expansion device 15. The inner side clamps 35 include displacement bodies 35a and fixed bodies 35b that are inserted into the notches 102, and by bringing the displacement bodies 35a in proximity to the fixed bodies 35b, close the predetermined flow paths 44, whereas by the displacement bodies 35a being made to separate away from the fixed bodies 35b, open the predetermined flow paths 44. In the flow paths 44 of the flow path opening/closing units 100, there are provided the aforementioned gas outflow route, the IC route 44A, a waste liquid route 44C from the EC route 44B to the waste liquid bag 18D, a collection route 44D from the IC route 44A to the collection bag 18E, and the second IC port path 44A2.

The target parameter detection parts 110 make it possible to detect parameters related to culturing of cells of the liquid that flows in the bioreactor 21. As the parameters related to culturing of cells, there may be cited, for example, an amount of dissolved oxygen, a pH, an amount of glucose, an amount of dissolved carbon dioxide, and an amount of lactic acid, of the liquid that flows in the flow paths 44. The amount of dissolved oxygen, the pH, and the amount of glucose correspond to the parameters of the culturing environment supplied to the cells. The amount of dissolved carbon dioxide and the amount of lactic acid correspond to parameters generated by metabolism of cells. One of the target parameter detection parts 110 makes it possible to detect any one of these five types of parameters.

More specifically, in the cell expansion system 23, the target parameter detection parts 110 provided in the cassette 10, and optical sensors 120 provided in the cell expansion device 15 jointly form culture parameter detection units 122 that detect parameters related to the cells at a time of cell culturing. The cell expansion system 23 (cell expansion device 15) adjusts (feeds back) the amounts of the culture medium and the gas component supplied to the bioreactor 21, on the basis of the detection results of the culture parameter detection units 122. Consequently, it becomes possible to appropriately manage the state of the cells (the number of cells, etc.) in the bioreactor 21.

According to the present embodiment, a plurality of (three) of the target parameter detection parts 110 are provided in the second EC port path 44B2, and are configured to detect different types of parameters. respectively. The three target parameter detection parts 110 are arranged alongside one another at equal intervals along the direction in which the second EC port path 44B2 extends. Moreover, at least one of the target parameter detection parts 110 may be provided in the cassette main body 40, or five of the target parameter detection parts 110 may be provided in order to detect all of the aforementioned five types of parameters. Further, the target parameter detection parts 110 are not limited to being disposed in the second EC port path 44B2, and may be disposed in another one of the flow paths 44 (for example, the second IC port path 44A2).

As shown in FIG. 5A, each of the target parameter detection parts 110 includes a fluorescent chip 112 (chip 111), and in the set state, the target parameter detection parts 110 are arranged respectively at positions facing toward the optical sensors 120. The fluorescent chips 112 are configured so as to undergo coloring in response to a predetermined substance (any one of oxygen, H⁺, OH⁻, glucose, carbon dioxide, or lactic acid) contained in the liquid. Under the control of a control unit 136 of the cell expansion device 15, each of the optical sensors 120 emits toward each of the fluorescent chips 112 measurement light having a wavelength corresponding to the characteristics of the fluorescent chip 112, and receives excitation light generated by excitation of the fluorescent chip 112. Consequently, the optical sensors 120 transmit to the control unit 136 detection signals based on the degree of coloration of the fluorescent chips 112.

More specifically, as shown in FIG. 5B, the target parameter detection parts 110 include the above-described fluorescent chips 112 disposed inside the flow path 44, and bulging portions 116 formed in the cassette main body 40 and in which the fluorescent chips 112 are accommodated. In the pair of resin sheets 42 that constitute the cassette main body 40, the fluorescent chips 112 are joined to a resin sheet 42a (on a side opposite from a resin sheet 42b on the side of the cover portion 54) which is in contact with a placement surface 15a of the cell expansion device 15.

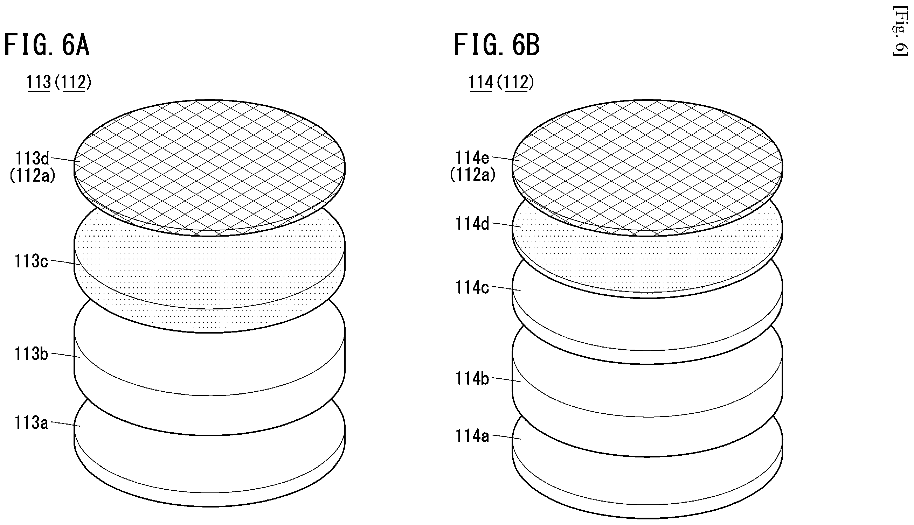

The fluorescent chips 112 may adopt an appropriate structure for the purpose of optically measuring the predetermined parameters (the amount of dissolved oxygen, the pH, the amount of glucose, the amount of dissolved carbon dioxide, the amount of lactic acid) related to culturing of cells. For example, FIG. 6A shows a laminated structure 113 of a first exemplary configuration of the fluorescent chip 112 for detecting a parameter, and FIG. 6B shows a laminated structure 114 of a second exemplary configuration of the fluorescent chip 112 for detecting a parameter.

The laminated structure 113 according to the first exemplary configuration is constituted by laminating or stacking four layers. More specifically, in sequential order from the side of an adhesive (the side of the resin sheet 42a), there are stacked an adhesive layer 113a, a polycarbonate layer 113b, a dye agent containing silicon layer 113c (detection layer), and a light shielding layer 113d. The adhesive layer 113a serves to adhere the laminated structure 113 to the resin sheet 42a. The polycarbonate layer 113b is a resin base material which is the largest in thickness among the four layers, and further is formed to possess translucency. In the polycarbonate layer 113b, a primer that fixes the dye agent containing silicon layer 113c may be applied to a surface thereof that faces toward the dye agent containing silicon layer 113c.

An appropriate dye agent (fluorescent substance) is applied to the dye agent containing silicon layer 113c depending on the parameter to be detected. For example, in the case of a configuration in which an amount of dissolved oxygen is detected as the parameter related to culturing of cells, benzo[ghi]perylene (polycyclic aromatic hydrocarbon) may be applied as the dye agent. The dye agent containing silicon layer 113c which is configured in such a manner undergoes coloring in proportion to the amount of dissolved oxygen contained in the liquid. Consequently, the dye agent containing silicon layer 113c outputs excitation light having a peak wavelength of 475 nm, for example, when measurement light having a peak wavelength of 400 nm is emitted from the optical sensors 120.

Alternatively, in the case of a configuration in which pH is detected as the parameter related to culturing of cells, pyranine (Pyranine: aryl sulfonate) can be applied as the dye agent. The dye agent containing silicon layer 113c which is configured in such a manner undergoes coloring in proportion to the amount of dissolved oxygen contained in the liquid. Consequently, the dye agent containing silicon layer 113c outputs excitation light having a peak wavelength of 555 nm, for example, when measurement light having a peak wavelength of 470 nm is emitted from the optical sensors 120.

Further, in the case of a configuration in which an amount of glucose is detected as the parameter related to culturing of cells, a reagent which primarily includes glucose oxidase (GOD), peroxidase (POD), glucose dehydrogenase (GDH), or the like can be applied as the dye agent.

Furthermore, in the case of a configuration in which an amount of dissolved carbon dioxide is detected as the parameter related to culturing of cells, similar to the case of detecting pH, pyranine can be applied as the dye agent.

Still further, in the case of a configuration in which an amount of lactic acid is detected as the parameter related to culturing of cells, lactate dehydrogenase (LDH), peroxidase (POD), or the like can be applied as the dye agent.

Further, as shown in FIG. 6B, the laminated structure 114 according to the second exemplary configuration is constituted by laminating or stacking five layers. In sequential order from the side of an adhesive (the side of the resin sheet 42a), the five layers are an adhesive layer 114a, a polycarbonate layer 114b, a urethane adhesive layer 114c, a sensitive membrane layer 114d (detection layer), and a light shielding layer 114e. In this case, a dye agent that reacts with a parameter included in the liquid is contained in the sensitive membrane layer 114d. As the dye agent, one of the above-describe dye agents can be applied, and for example, in the case of a structure for detecting pH, pyranine is preferably applied.

As shown in FIGS. 6A and 6B, each of the fluorescent chips 112 (the laminated structures 113 and 114) is provided with a light shielding layer 112a (the light shielding layers 113d and 114e) at a protruding end thereof, whereby leakage of measurement light to the surrounding vicinity of the fluorescent chips 112 is suppressed. Furthermore, returning to FIG. 5B, the light shielding layer 112a may have a light blocking coating 112a1 thereon that covers the side peripheral surfaces of the fluorescent chips 112. The light blocking coating 112al includes a portion that connects the dye agent containing silicon layer 113c or the sensitive membrane layer 114d and the flow path 44, and covers other side peripheral surfaces to thereby further suppress leakage of light from the fluorescent chips 112.

On the other hand, the bulging portions 116 of the target parameter detection parts 110 are portions in which the pair of resin sheets 42 bulge in a direction perpendicular to the planar direction of the cassette main body 40 (the direction in which the flow paths 44 extend), and include space portions 116a that communicate with the flow path 44 on the inner side thereof. The flow path cross-sectional area of the space portions 116a is set to be sufficiently greater than the flow path cross-sectional area of the flow paths 44, and the liquid is capable of flowing satisfactorily even in a state in which the fluorescent chips 112 are accommodated therein.

Each of the bulging portions 116 in the pair of resin sheets 42 includes a flat portion 117, and inclined portions 118 disposed on the outer periphery of the flat portion 117, as viewed in cross-section along a thickness direction of the cassette main body 40. The fluorescent chips 112 are fixed to the flat portions 117 of the resin sheet 42a, and in the set state, the flat portions 117 are configured so as to contact the optical sensors 120. Moreover, the inclined portions 118 of the resin sheet 42a may be provided with shielding regions 118a, in order to suppress leakage of the measurement light of the optical sensors 120.

Further, the optical sensors 120, and placement recesses 124 are provided in portions in the cell expansion device 15 that constitute the culture parameter detection units 122. Each of the optical sensors 120 includes a light emitting unit 120a that emits measurement light having a predetermined wavelength, and a light receiving unit 120b that receives excitation light generated from the fluorescent chips 112, on the basis of the configuration (parameters) of the fluorescent chips 112.

The placement recesses 124 are formed with a predetermined depth (a dimension shorter than the protruding amount of the bulging portions 116) from the placement surface 15a of the cell expansion device 15, and include the optical sensors 120 at bottom parts thereof. When the cassette 10 is set, the placement recesses 124 are capable of guiding the bulging portions 116 of the cassette main body 40 (the resin sheet 42a), whereby the flat portions 117 are placed on and brought into contact with the bottom parts thereof. Consequently, the optical sensors 120 are capable of stably detecting the parameters of the fluorescent chips 112.

Returning to FIG. 1, the cell expansion device 15 in which the kit 12 is mounted is equipped with a box-shaped device main body 130, and a stand 132 on which the medical bags 18 of the kit 12 are retained. Further, a touch panel 134 (display operation unit) for carrying out operations and displays when the cell expansion process is performed is provided on an outer surface of the device main body 130. Furthermore, in the interior of the device main body 130, there are provided a cassette placement unit (not shown) in which the cassette 10 is fixed in an upright posture, and further, the bioreactor 21 is retained at an appropriate height, and the above-described control unit 136 that controls operation of the cell expansion system 23. Although illustration thereof is omitted, it goes without saying that the cell expansion device 15 may include a functional unit for realizing various conditions that are required for culturing of cells. For example, the cell expansion device 15 may be equipped with a temperature adjustment unit that executes a temperature control to maintain the culturing environment at 37°C.

The control unit 136 includes a non-illustrated processor, a memory, and an input/ output interface, and by the processor executing a program stored in the memory, in the expansion process, the pumps 30, the outer side clamps 34, the inner side clamps 35, etc., are appropriately operated. Further, the control unit 136 receives detection signals from each of the optical sensors 120 under the execution of the program at the time of cell culturing, and adjusts (feedback controls) driving of the pumps 30 on the basis of the detection results thereof.

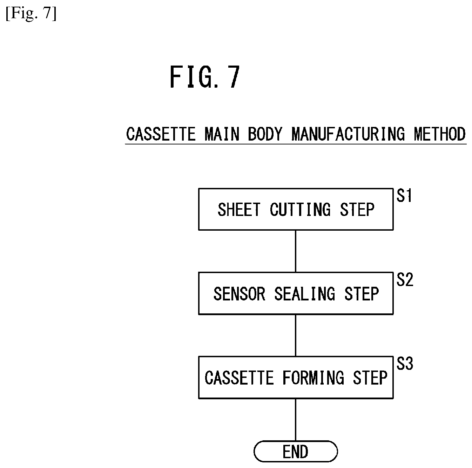

Next, a description will be given concerning a manufacturing procedure (cassette main body manufacturing method) for the cassette main body 40 having the above-described fluorescent chips 112. In such a manufacturing process, as shown in FIG. 7, a sheet cutting step (step S1), a sensor sealing step (step S2), and a cassette forming step (step S3) are sequentially performed.

In the sheet cutting step, using a non-illustrated cutting device, a uniformly continuous base material (not shown) for the sheets is cut according to the shape and size of the cassette main body 40 to be manufactured, whereby the resin sheets 42 for each one of the sheets are formed.

In the sensor sealing step, using a non-illustrated adhering device, the separately formed fluorescent chips 112 (the laminated structures 113 and 114) are sealed at predetermined positions (intended formation positions where the target parameter detection parts 110 are to be formed) of one of the obtained resin sheets 42a. As noted previously, the fluorescent chips 112 include the adhesive layers 113a and 114a, and the fluorescent chips 112 can be easily and quickly adhered accompanying the positioning of the resin sheet 42a.

In the cassette forming step, the resin sheet 42a to which the fluorescent chips 112 are fixed, and the resin sheet 42b are superposed on a mold having grooves therein for forming the flow paths 44 (including the target parameter detection parts 110, etc.). In addition, while the mold sandwiches the pair of resin sheets 42 and fusion bonding is performed, air is supplied and discharged between the pair of resin sheets 42 corresponding to the grooves, whereby the flow paths 44 are formed. At this time, the bulging portions 116 are formed while the fluorescent chips 112 are fixed to the resin sheet 42a, and after having performed the cassette forming step, the cassette main body 40 develops a configuration in which the target parameter detection parts 110 are included.

Next, a description will be given concerning operations of the tubes 16 and the flow paths 44 of the cassette 10 in the cell expansion process of the cell expansion system 23.

As shown in FIG. 1, in the expansion process of the cell expansion system 23, an operator inserts portions of the kit 12 including the cassette 10 into the cell expansion device 15. Further, the operator places the appropriate tubes 16 of the kit 12 on the pumps 30, the air bubble sensor 32, and the outer side clamps 34 of the cell expansion device 15. Furthermore, when the cassette 10 is arranged, the flow path opening/ closing units 100 are arranged in the inner side clamps 35, and the target parameter detection parts 110 are arranged in the optical sensors 120 (the placement recesses 124). Consequently, as shown in FIG. 3, the cassette 10 is set in the cell expansion device 15 with the planar direction thereof being oriented in a posture along the direction of gravity. Furthermore, the medical bags 18 of the kit 12 are also suspended from the stand 132 by the operator.

After having been set, in the expansion process, a priming step, a culture medium replacement step, a seeding step, a culturing step, a releasing step, and a collecting step are sequentially performed. In the priming step, the cleaning solution in the cleaning solution bag 18B is made to flow through the two routes (the IC route 44A and the EC route 44B) inside the cassette 10, and is guided into the waste liquid bag 18D, while the gas existing in the respective routes of the predetermined tubes 16, the bioreactor 21, and the cassette main body 40 is removed. In the culture medium replacement step, in the same manner as in the priming step, the culture medium in the culture medium bag 18C is guided into the respective routes of the predetermined tubes 16, the bioreactor 21, and the cassette main body 40.

Furthermore, in the seeding step, after having performed the culture medium replacement step, the cell solution of the cell solution bag 18A is supplied via the IC route 44A to the inner cavities of the hollow fibers 24 of the bioreactor 21, while the culture medium existing in the EC route 44B is circulated and the gas component is supplied to the bioreactor 21.

In addition, in the culturing step after having performed the seeding step, as shown in FIG. 4, the culture medium is supplied from both the IC route 44A and the EC route 44B, and culturing of the cells that were seeded in the bioreactor 21 is carried out. The culturing step is carried out for a longer period of time (for example, over several days) in comparison with the other steps, whereby the cells on the inner peripheral surfaces of the hollow fibers 24 are made to expand. Moreover, in the cell expansion system 23, an operation of supplying the culture medium from the EC route 44B without using the IC route 44A may be carried out in the culturing step.

When the culturing step is performed, the cell expansion system 23 detects parameters related to culturing of cells by the culture parameter detection units 122 (the target parameter detection parts 110 and the optical sensors 120), and controls driving of the pumps 30 in accordance with the detection results. In the culturing step, the culture medium flowing out from the bioreactor 21 flows in the second EC port path 44B2, and the target parameter detection parts 110 (the fluorescent chips 112) undergo coloring by reacting with target substances contained in the culture medium. The optical sensors 120 emit measurement light toward the fluorescent chips 112, receive excitation light of the colored fluorescent chips 112, and transmit detection signals (information on absorbance) based on the received light to the control unit 136.

For example, in a configuration for detecting the amount of dissolved oxygen, in the case that the amount of dissolved oxygen is large, the control unit 136 reduces the supplied amount of oxygen (i.e., the rate at which the culture medium is circulated on the side of the EC route 44B), whereas in the case that the amount of dissolved oxygen is small, the control unit 136 increases the supplied amount of oxygen. Further, in a configuration for detecting pH, in the case that the pH is high, the control unit 136 increases the supplied amount of carbon dioxide (i.e., the rate at which the culture medium is circulated on the side of the EC route 44B), whereas in the case that the pH is low, the control unit increases the supplied amount of the culture medium (the amount of the culture medium supplied to the IC route 44A or the EC route 44B). Furthermore, in a configuration for detecting the amount of glucose, in the case that the amount of glucose is high, the control unit 136 reduces the supplied amount of the culture medium, whereas in the case that the amount of glucose is low, the control unit 136 increases the supplied amount of the culture medium.

On the other hand, in a configuration for detecting the amount of dissolved carbon dioxide, in the case that the amount of dissolved carbon dioxide is large, the control unit 136 reduces the supplied amount of the culture medium. Conversely, in the case that the amount of dissolved carbon dioxide is small, the control unit 136 increases the supplied amount of the culture medium. Similarly, in a configuration for detecting the amount of lactic acid, also in the case that the amount of lactic acid is high, the control unit 136 reduces the supplied amount of the culture medium. Conversely, in the case that the amount of lactic acid is small, the control unit 136 increases the supplied amount of the culture medium.

In a configuration in which a plurality of the culture parameter detection units 122 detect a plurality of types of parameters, the control unit 136 optimizes driving of the pumps 30 by comparing and correcting the different types of parameters. Consequently, the cell expansion system 23 can appropriately manage the expansion of cells in the bioreactor 21.

Further, in the releasing step after having performed the culturing step, the releasing solution is supplied from the IC route 44A to thereby release the cells that were cultured (expanded) inside the bioreactor 21. In the releasing step, a medium containing a gas component is circulated between the EC route 44B and the bioreactor 21. Further, in the collecting step after having performed the releasing step, by supplying the culture medium to the IC route 44A, the cells that were released in the releasing step are made to flow out from the bioreactor 21 and are guided into the collection bag 18E. At this time, the culture medium and the gas component are also supplied through the EC route 44B.

By the aforementioned step, the cell expansion system 23 can satisfactorily store the cells that were cultured in the bioreactor 21 in the collection bag 18E. Moreover, the detection of the parameters related to culturing of cells by the target parameter detection parts 110 is not limited to being performed in the culturing step, and can naturally be performed in other steps.

Further, the present invention is not limited to the above-described embodiment, and various modifications can be adopted in accordance with the essence and gist of the present invention. For example, the cell expansion system 23 may detect as a parameter the temperature of the liquids that flow through the flow paths 44 of the cassette main body 40, and a flow control for the liquids may be carried out while taking into consideration the above-described parameters (the amount of dissolved oxygen, the pH, the amount of glucose, the amount of dissolved carbon dioxide, and the amount of lactic acid), and the detected temperature.

Technical concepts and effects that can be grasped from the above-described embodiments will be described below.

A first aspect of the present invention is the biological component cassette 10 having therein the flow paths 44 through which the liquids for culturing cells are allowed to flow, and including the cassette main body 40 formed in a sheet shape that possesses flexibility, wherein in the flow paths 44, there is provided the target parameter detection part 110 which makes it possible to detect a parameter related to culturing of cells, and the target parameter detection part 110 includes the chip 111 configured to undergo coloring in response to a predetermined substance contained in the liquids.

In accordance with such features, when the biological component cassette 10 is set by an operator in the biological component treatment device 14, it is possible to easily and accurately arrange the plurality of paths, and enable setting to be carried out efficiently. In addition, the biological component cassette 10 includes the target parameter detection part 110 (the chip 111) in the flow paths 44 of the cassette main body 40, whereby the parameters related to culturing of cells can be easily detected. As a result, in the biological component cassette 10, it is possible to satisfactorily adjust the state of the culture medium and the gas component at the time of cell culturing based on the parameters related to culturing of cells, and product quality can be stabilized.

Further, the target parameter detection part 110 is capable of detecting, as the parameter related to culturing of cells, one parameter from among an amount of dissolved oxygen, a pH, an amount of glucose, an amount of dissolved carbon dioxide, and an amount of lactic acid. In accordance with this feature, the target parameter detection part 110 is capable of detecting a target parameter (any one from among an amount of dissolved oxygen, a pH, an amount of glucose, an amount of dissolved carbon dioxide, and an amount of lactic acid) with higher accuracy.

Further, the cassette main body 40 includes a plurality of the target parameter detection parts 110, and the plurality of target parameter detection parts 110 are capable of detecting different types of parameters. In accordance with this feature, the biological component cassette 10 can be made to execute the control in greater detail at the time of cell culturing, and product quality can be enhanced.

Further, the biological component cassette 10 is connected to the bioreactor 21 in which the cells are cultured, and causes the liquid to flow out therefrom and supplies the liquid to the bioreactor 21, and the liquid discharged from the bioreactor 21 flows into the biological component cassette, and the target parameter detection part 110 is provided in a port path (the second IC port path 44A2, the second EC port path 44B2), within the flow path 44, into which the liquid from the bioreactor 21 flows. In accordance with such features, the biological component cassette 10 can satisfactorily detect the state of the liquid discharged from the bioreactor 21, and is capable of further stabilizing product quality.

The target parameter detection part 110 includes the bulging portion 116 that protrudes in a direction perpendicular to a planar direction of the cassette main body 40, the bulging portion being configured to accommodate the chip 111 therein. In accordance with this feature, even with a configuration in which the chip 111 is provided in the flow paths 44, the biological component cassette 10 can detect the parameter related to culturing of cells while the liquid is allowed to smoothly flow.

Further, the cassette main body 40 is constituted by joining together the pair of resin sheets 42, and the chip 111 is adhered to one of the pair of resin sheets 42 and protrudes inside the flow path 44 in a direction perpendicular to the direction in which the flow path 44 extends, the chip being configured as the laminated structure 113 or 114 including the detection layer (the dye agent containing silicon layer 113c, the sensitive membrane layer 114d) that undergoes coloring based on the predetermined substance. In accordance with such features, when measurement light enters from one of the pair of resin sheets 42, the chip 111 can suitably guide the measurement light to the detection layer that has undergone coloring, and can return the excitation light.

Further, the light shielding layer 113d or 114e that blocks leakage of light is provided on the protruding end of the chip 111. In accordance with this feature, when optical measurement is performed on the chip 111, the target parameter detection part 110 can suppress leakage of light from the protruding end of the chip 111, and can accurately detect the parameter.

Further, the light blocking coating 112al that blocks leakage of light is provided on a side surface of the chip 111. In accordance with this feature, when optical measurement is performed on the chip 111, the target parameter detection part 110 can suppress leakage of light from the side surface of the chip 111, and can accurately detect the parameter.

Further, a second aspect of the present invention is the biological component kit 12 including the tubes 16 through which the liquids for culturing cells are allowed to flow, and the biological component cassette 10 to which the tubes 16 are connected, wherein the biological component cassette 10 includes the flow paths 44 in the interior thereof through which the liquids are allowed to flow, and includes the cassette main body 40 formed in a sheet shape that possesses flexibility, in the flow paths 44, there is provided the target parameter detection part 110 which makes it possible to detect a parameter related to culturing of cells, and the target parameter detection part 110 includes the chip 111 that undergoes coloring in response to a predetermined substance contained in the liquid. In accordance with such features, the biological component kit 12 can easily and accurately arrange the plurality of paths, and enable setting to be carried out efficiently. Further, in the biological component kit 12, it is possible to easily detect the parameters related to culturing of cells, and to adjust the state of the culture medium and the gas component at the time of cell culturing, and product quality can be stabilized.

Further, a third aspect of the present invention is the biological component treatment system 22, including the biological component kit 12 including the tubes 16 through which the liquids for culturing cells are allowed to flow, and the biological component cassette 10 to which the tubes 16 are connected, and the biological component treatment device 14 in which the biological component kit 12 is set, wherein the biological component cassette 10 includes the flow paths 44 in the interior thereof through which the liquids are allowed to flow, and includes a cassette main body 40 formed in a sheet shape that possesses flexibility, in the flow paths 44, there is provided the target parameter detection part 110 through which a parameter related to culturing of cells is detectable, the target parameter detection part 110 includes the chip 111 that undergoes coloring in response to a predetermined substance contained in the liquid, and the biological component treatment device 14 includes an optical sensor 120 configured to detect the parameter related to culturing of cells, by emitting measurement light toward the chip 111 and receiving excitation light from the chip 111. In accordance with such features, in the biological component treatment system 22, setting can be carried out efficiently, and it is possible to stabilize product quality by enabling easy detection of the parameters related to culturing of cells.

Further, the target parameter detection part 110 includes the bulging portion 116 that protrudes in a direction perpendicular to the planar direction of the cassette main body 40, the bulging portion being configured to accommodate the chip 111 therein, and the biological component treatment device 14 includes the placement recess 124 in which the bulging portion 116 is arranged, and the optical sensor 120 is disposed in a bottom part of the placement recess 124. In accordance with such features, in the biological component treatment system 22, when the biological component cassette 10 is set in the biological component treatment device 14, the target parameter detection part 110 can be smoothly arranged in the placement recess 124. Further, in the set state, the optical sensor 120 faces toward the chip 111, and it becomes possible to satisfactorily carry out optical measurement of the parameter related to culturing of cells.

Moreover, the chips 111 that are applied to the cassette 10 (the target parameter detection parts 110) are not limited to the fluorescent chips 112 having fluorescent substances that absorb light of a predetermined wavelength and emit excitation light of a different wavelength, and chips 111 in accordance with various optical measurement methods may be applied thereto. For example, for the chips 111, there may be applied chips that are made to change a color tone in accordance with the concentration of a predetermined substance (parameter). In this case, for the optical sensors 120, there can be adopted a measurement method in which measurement light is emitted toward the chips 111, and reflected light therefrom is measured.

## Claims

1. A biological component cassette (10) having therein a flow path (44) through which a liquid for culturing cells is allowed to flow, and comprising a cassette main body (40) formed in a sheet shape, wherein:
in the flow path (44), there is provided a target parameter detection part (110) through which a parameter related to culturing of cells is detectable; and
**characterized in that**
the cassette main body (40) formed in a sheet shape possesses flexibility,
the target parameter detection part (110) includes a chip (111) configured to undergo coloring in response to a predetermined substance contained in the liquid, wherein
the target parameter detection part (110) includes a bulging portion (116) that protrudes in a direction perpendicular to a planar direction of the cassette main body (40), the bulging portion (116) being configured to accommodate the chip (111) therein.

2. The biological component cassette (10) according to claim 1, wherein the target parameter detection part (110) is configured to detect, as the parameter related to culturing of cells, one parameter from among an amount of dissolved oxygen, a pH, an amount of glucose, an amount of dissolved carbon dioxide, and an amount of lactic acid.

3. The biological component cassette (10) according to claim 1 or 2, wherein:
the cassette main body (40) includes a plurality of the target parameter detection parts (110); and
the plurality of target parameter detection parts (110) are configured to detect different types of parameters.

4. The biological component cassette (10) according to any one of claims 1 to 3, wherein:
the biological component cassette (10) is connected to a bioreactor (21) configured to culture the cells, and causes the liquid to flow out therefrom and supplies the liquid to the bioreactor, and the liquid discharged from the bioreactor (21) flows into the biological component cassette (10); and
the target parameter detection part (110) is provided in a port path, within the flow path (44), into which the liquid from the bioreactor (21) flows.

5. The biological component cassette (10) according to any one of claims 1 to 4, wherein
the cassette main body (40) is constituted by joining together a pair of resin sheets (42); and
the chip (111) is adhered to one of the pair of resin sheets (42) and protrudes inside the flow path (44) in a direction perpendicular to a direction in which the flow path (44) extends, the chip (111) being configured as a laminated structure comprising a detection layer containing a fluorescent substance that undergoes coloring based on the predetermined substance.

6. The biological component cassette (10) according to claim 5, wherein a light shielding layer configured to block leakage of light is provided on a protruding end of the chip (111).

7. The biological component cassette (10) according to claim 6, wherein a light blocking coating configured to block leakage of light is provided on a side surface of the chip (111).

8. A biological component kit comprising:
a tube through which a liquid for culturing cells is allowed to flow; and
a biological component cassette (10) according to any of claims 1 to 7.

9. A biological component treatment system, comprising:
a biological component kit including a tube through which a liquid for culturing cells is allowed to flow, and a biological component cassette (10) to which the tube is connected; and
a biological component treatment device in which the biological component kit is set;
wherein the biological component cassette (10) is the biological component cassette according to any of claims 1 to 7; and
the biological component treatment device includes an optical sensor configured to detect the parameter related to culturing of cells, by emitting measurement light toward the chip (111) and receiving light from the chip (111).

10. The biological component treatment system according to claim 9, wherein:
the biological component treatment device comprises a placement recess in which the bulging portion (116) is arranged, and the optical sensor is disposed in a bottom part of the placement recess.

## Patentansprüche

1. Kassette (10) für biologische Komponenten mit einem darin ausgebildeten Strömungspfad (44), durch den eine Flüssigkeit zur Zellkultivierung fließen kann, und mit einem in Folienform ausgebildeten Kassettenhauptkörper (40), wobei:
im Strömungspfad (44) ein Teil (110) zur Erfassung von Zielparametern bereitgestellt ist, durch den ein für die Zellkultivierung betreffender Parameter erfasst wird; und
**dadurch gekennzeichnet, dass**
der in Form einer Folie ausgebildete Kassettenhauptkörper (40) flexibel ist,
der Erfassungsabschnitt (110) für den Zielparameter einen Chip (111) hat, der so konfiguriert ist, dass er sich als Reaktion auf eine in der Flüssigkeit enthaltene vorbestimmte Substanz verfärbt, wobei
der Erfassungsabschnitt (110) für den Zielparameter einen sich wölbenden Abschnitt (116) hat, der in einer Richtung senkrecht zur flächigen Ebene des Kassettenhauptkörpers (40) vorragt, wobei der sich wölbende Abschnitt (116) so konfiguriert ist, dass er den Chip (111) in sich aufnimmt.

2. Kassette (10) für biologische Komponenten gemäß Anspruch 1, wobei der Erfassungsabschnitt (110) für den Zielparameter konfiguriert ist, dass er als den Parameter, der die Zellkultivierung betrifft, einen Parameter aus der Gruppe bestehend aus der Menge an gelöstem Sauerstoff, dem pH-Wert, der Glukosemenge, der Menge an gelöstem Kohlendioxid und der Menge an Milchsäure erfasst.

3. Kassette (10) für biologische Komponenten gemäß Anspruch 1 oder 2, wobei:
der Kassettenhauptkörper (40) eine Vielzahl von Erfassungsabschnitten (110) für Zielparameter hat; und
die Vielzahl von Erfassungsabschnitten (110) für Zielparameter so konfiguriert ist, dass sie verschiedene Arten von Parametern erfasst.

4. Die Kassette (10) für biologische Komponenten gemäß einem der Ansprüche 1 bis 3, wobei:
die Kassette (10) für biologische Komponenten mit einem Bioreaktor (21) verbunden ist, der zur Kultivierung der Zellen konfiguriert ist, und bewirkt, dass die Flüssigkeit aus diesem abfließt, und die Flüssigkeit dem Bioreaktor zuführt, wobei die aus dem Bioreaktor (21) abgegebene Flüssigkeit in die Kassette (10) für biologische Komponenten fließt; und
der Nachweisteil (110) für den Zielparameter in einem Anschluss innerhalb des Strömungspfads (44) bereitgestellt ist, in den die Flüssigkeit aus dem Bioreaktor (21) fließt.

5. Kassette (10) für biologische Komponenten gemäß einem der Ansprüche 1 bis 4, wobei
der Kassettenhauptkörper (40) durch Zusammenfügen eines Paares von Kunststofffolien (42) ausgebildet ist; und
der Chip (111) an einer der beiden Kunststofffolien (42) befestigt ist und in den Strömungspfad (44) in einer Richtung senkrecht zur Ausdehnungsrichtung des Strömungspfads (44) vorragt, wobei der Chip (111) als laminierte Struktur konfiguriert ist, die eine Detektionsschicht hat, die eine fluoreszierende Substanz enthält, die beruhend auf der vorbestimmten Substanz eine Farbänderung durchläuft.

6. Kassette (10) für biologische Komponenten nach Anspruch 5, wobei an einem vorstehenden Ende des Chips (111) eine Lichtabschirmschicht bereitgestellt ist, die so konfiguriert ist, dass sie das Austreten von Licht verhindert.

7. Kassette (10) für biologische Komponenten gemäß Anspruch 6, wobei an einer Seitenfläche des Chips (111) eine lichtblockierende Beschichtung bereitgestellt ist, die so ausgebildet ist, dass sie das Austreten von Licht verhindert.

8. Kit für biologische Komponenten, mit:
einem Schlauch, durch den eine Flüssigkeit zur Zellkultivierung fließen kann; und
einer biologischen Komponentenkassette (10) gemäß einem der Ansprüche 1 bis 7.

9. System zur Behandlung biologischer Komponenten, mit:
einem Kit für biologische Komponenten, das einen Schlauch hat, durch den eine Flüssigkeit zur Zellkultivierung fließen kann, und einer Kassette (10) für biologische Komponenten, mit der dem Schlauch verbunden ist; und
einer Behandlungsvorrichtung für biologische Komponenten, in die das Kit für biologische Komponenten eingesetzt ist;
wobei die Kassette (10) für biologische Komponenten die Kassette für biologische Komponenten gemäß einem der Ansprüche 1 bis 7 ist; und
die Vorrichtung zur Behandlung biologischer Komponenten einen optischen Sensor hat, der so konfiguriert ist, dass er den für die Zellkultur relevanten Parameter erfasst, indem er ein Messlicht in Richtung des Chips (111) aussendet und Licht vom Chip (111) empfängt.

10. System zur Behandlung biologischer Komponenten gemäß Anspruch 9, wobei:
die Vorrichtung zur Behandlung biologischer Komponenten eine Aufnahmeaussparung hat, in der der sich wölbende Abschnitt (116) angeordnet ist, und der optische Sensor in einem unteren Teil der Aufnahmeaussparung angeordnet ist.

## Revendications

1. Cassette de composant biologique (10) comportant un trajet d'écoulement (44) à travers lequel un liquide pour la culture de cellules peut s'écouler, et comprenant un corps principal de cassette (40) formé en forme de feuille, dans laquelle :
dans le trajet d'écoulement (44), il est prévu une partie de détection de paramètre cible (110) à travers laquelle un paramètre lié à la culture de cellules est détectable ; et
**caractérisée en ce que**
le corps principal de cassette (40) formé en forme de feuille possède une flexibilité,
la partie de détection de paramètre cible (110) inclut une puce (111) configurée pour subir une coloration en réponse à une substance prédéterminée contenue dans le liquide, dans laquelle
la partie de détection de paramètre cible (110) inclut une partie bombée (116) qui fait saillie dans une direction perpendiculaire à une direction plane du corps principal de cassette (40), la partie bombée (116) étant configurée pour recevoir la puce (111) dans celle-ci.

2. Cassette de composant biologique (10) selon la revendication 1, dans laquelle la partie de détection de paramètre cible (110) est configurée pour détecter, en tant que paramètre lié à une culture de cellules, un paramètre parmi une quantité d'oxygène dissous, un pH**,** une quantité de glucose, une quantité de dioxyde de carbone dissous et une quantité d'acide lactique.

3. Cassette de composant biologique (10) selon la revendication 1 ou 2, dans laquelle :
le corps principal de cassette (40) inclut une pluralité de parties de détection de paramètre cible (110) ; et
la pluralité de parties de détection de paramètre cible (110) sont configurées pour détecter différents types de paramètres.

4. Cassette de composant biologique (10) selon l'une quelconque des revendications 1 à 3, dans laquelle :
la cassette de composant biologique (10) est reliée à un bioréacteur (21) configuré pour une culture des cellules, et provoque l'écoulement du liquide hors de celle-ci et alimente le liquide au bioréacteur, et le liquide évacué du bioréacteur (21) s'écoule dans la cassette de composant biologique (10) ; et
la partie de détection de paramètre cible (110) est disposée dans un passage d'orifice, à l'intérieur du trajet d'écoulement (44), dans lequel le liquide provenant du bioréacteur (21) s'écoule.

5. Cassette de composant biologique (10) selon l'une quelconque des revendications 1 à 4, dans laquelle
le corps principal de cassette (40) est constitué par assemblage d'une paire de feuilles de résine (42) ; et
la puce (111) est collée à l'une de la paire de feuilles de résine (42) et fait saillie à l'intérieur du trajet d'écoulement (44) dans une direction perpendiculaire à une direction dans laquelle s'étend le trajet d'écoulement (44), la puce (111) étant configurée comme une structure stratifiée comprenant une couche de détection contenant une substance fluorescente qui subit une coloration basée sur la substance prédéterminée.

6. Cassette de composant biologique (10) selon la revendication 5, dans laquelle une couche pare-lumière configurée pour bloquer une fuite de lumière est fournie sur une extrémité saillante de la puce (111).

7. Cassette de composant biologique (10) selon la revendication 6, dans laquelle un revêtement de blocage de lumière configuré pour bloquer une fuite de lumière est fourni sur une surface latérale de la puce (111).

8. Kit de composant biologique comprenant :
un tube à travers lequel un liquide pour une culture de cellules peut s'écouler ; et
une cassette de composant biologique (10) selon l'une quelconque des revendications 1 à 7.

9. Système de traitement de composant biologique, comprenant :
un kit de composant biologique incluant un tube à travers lequel un liquide pour une culture de cellules peut s'écouler, et une cassette de composant biologique (10) à laquelle le tube est relié ; et
un dispositif de traitement de composant biologique dans lequel le kit de composant biologique est placé ;
dans lequel la cassette de composant biologique (10) est la cassette de composant biologique selon l'une quelconque des revendications 1 à 7 ; et
le dispositif de traitement de composant biologique inclut un capteur optique configuré pour détecter le paramètre lié à une culture de cellules, en émettant une lumière de mesure vers la puce (111) et en recevant de la lumière de la puce (111).

10. Système de traitement de composant biologique selon la revendication 9, dans lequel :
le dispositif de traitement de composant biologique comprend un évidement de placement dans lequel est disposée la partie bombée (116), et le capteur optique est disposé dans une partie inférieure de l'évidement de placement.
